(19) Europäisches Patentamt
European Patent Office
Office européen des brevets

(11) Publication number: 0 433 043 A1

# (12) EUROPEAN PATENT APPLICATION

(21) Application number: 90313508.5

(22) Date of filing: 12.12.90

(51) Int. Cl.[5]: A61K 31/415, //(A61K31/415, 31:40)

(30) Priority: 13.12.89 GB 8928208

(43) Date of publication of application: 19.06.91 Bulletin 91/25

(84) Designated Contracting States: AT BE CH DE DK ES FR GB GR IT LI LU NL SE

(71) Applicant: GLAXO GROUP LIMITED
Clarges House 6-12 Clarges Street
London W1Y 8DH(GB)

(72) Inventor: Tyers, Michael Brian c/o Glaxo
Group Research
Park Road Ware
Hertfordshire(GB)

(74) Representative: Kyle, Diana et al
ELKINGTON AND FIFE Beacon House 113
Kingsway
London WC2B 6PP(GB)

## (54) Medicaments.

(57) Treatment of conditions associated with cephalic pain such as migraine, cluster headache, chronic paroxysmal hemicrania and headache associated with vascular disorders using 3-[2-(dimethylamino)ethyl]-N-methyl-1H-indole-5-methanesulphonamide in conjunction with a compound having antagonist activity at $5HT_3$ receptors, and pharmaceutical compositions containing 3-[2-(dimethylamino)ethyl]-N-methyl-1H-indole-5-methanesulphonamide together with a compound having antagonist activity at $5HT_3$ receptors.

EP 0 433 043 A1

Xerox Copy Centre

## MEDICAMENTS

This invention relates to the treatment of conditions associated with cephalic pain such as migraine, cluster headache, chronic paroxysmal hemicrania and headache associated with vascular disorders. In particular it relates to the use of 3-[2-(dimethylamino)ethyl]-N-methyl-1H-indole-5-methanesulphonamide in conjunction with a compound having antagonist activity at $5HT_3$ receptors in the treatment of such conditions and to pharmaceutical compositions containing the two compounds.

3-[2-(Dimethylamino)ethyl]-N-methyl-1H-indole-5-methanesulphonamide, which may be represented by the formula (I)

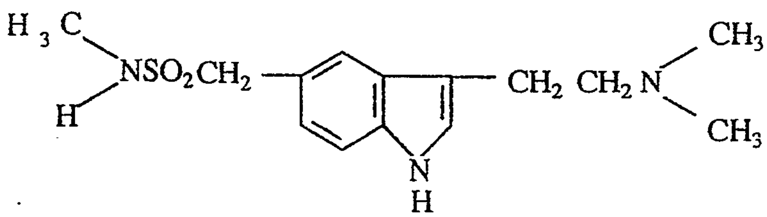

and its physiologically acceptable salts and solvates are disclosed in our UK Patent Specification No. 2162522. Compound (I) is a selective $5HT_1$-like receptor agonist and exhibits selective vasoconstrictor activity. It has been shown to be useful in the treatment of migraine and related disorders such as cluster headache. Other conditions associated with cephalic pain for which compounds, such as compound (I), which are selective $5HT_1$-like receptor agonists and which exhibit selective vasoconstrictor activity are useful include chronic paroxysmal hemicrania and headache associated with vascular disorders.

We have now found that administration of compound (I) in conjunction with a compound having antagonist activity at $5HT_3$ receptors is beneficial in the treatment of conditions associated with cephalic pain and in alleviating the symptoms associated therewith.

According to one aspect of the invention we therefore provide a method of treating conditions associated with cephalic pain and alleviating the symptoms associated therewith which comprises administering to a mammal, including man, compound (I) or a physiologically acceptable salt or solvate thereof and a compound having antagonist activity at $5HT_3$ receptors or a physiologically acceptable salt or solvate thereof.

It will be appreciated that reference to treatment is intended to include prophylaxis as well as the alleviation of established symptoms.

According to another aspect of the invention we provide the use of compound (I) or a physiologically acceptable salt or solvate thereof and a compound having antagonist activity at $5HT_3$ receptors or a physiologically acceptable salt or solvate thereof for the manufacture of a medicament for the treatment of conditions associated with cephalic pain and the alleviation of symptoms associated therewith.

Suitable physiologically acceptable salts of the compound of formula (I) for use according to the invention include acid addition salts formed with inorganic acids such as hydrochlorides, hydrobromides, phosphates and sulphates and with organic acids, for example tartrates, maleates, fumarates, succinates and sulphonates. A preferred form of the compound of formula (I) for use according to the invention is the succinate, most preferably the 1:1 succinate.

It will be appreciated that the present invention relates to the use of compound (I) in conjunction with any compound having antagonist activity at $5HT_3$ receptors known in the art.

A variety of compounds which act as antagonists of 5-HT at $5\text{-}HT_3$ receptors have been described in the art. These compounds are generally azabicyclo derivatives and/or benzoic acid derivatives or imidazole derivatives. The azabicyclo derivatives include compounds containing a bridged piperidyl group, such as tropyl, pseudotropyl, homotropyl or quinuclidinyl group, and they preferably contain a carbocyclic or heterocyclic aromatic group joined to the azabicyclic ring for example by an ester or amide link. The aromatic group may be for example an optionally substituted phenyl, indolyl, benzofuranyl, benzothienyl, benzisoxazolyl, indazolyl or pyrimidinyl group. The benzoic acid derivatives which act as antagonists of 5-HT at $5\text{-}HT_3$ receptors include benzoates and benzamides, for example esters or amides formed with an azabicyclic group as defined above, or formed with a piperidyl group.

Such compounds have been disclosed inter alia in published UK Patent Applications Nos. 2100259, 2125398, 2131420, 21321889, 2145416, 2152049, 215821 and 2169292, published European Patent

Applications Nos. 111608, 116255, 158265, 191562, 210840, 214772, 21913, 221702, 226267, 227215, 230718, 235878 and 242973, and published Australian Patent Application No. 87/67121. The compounds disclosed in published European Patent Applications Nos. 13138, 67615 and 94742 have also been described as antagonists of 5-HT at 5-$HT_3$ receptors, in published European Patent Applications Nos. 215545 and 220011. In addition, 4-amino-N-1-azabicyclo[2.2.2]oct-3-yl-5-chloro-2-methoxybenzamide (also known as zacopride), described in European Patent Specification No. 99789, has also now been shown to be an antagonist of 5-HT at $5HT_3$ receptors.

Preferred classes of $5HT_3$ receptor antagonists for use according to the present invention include 3-(imidazol-1-yl) methyltetrahydrocarbazolones, 3-(imidazol-4-yl)-1-(indol-3-yl)-1-propanones, azabicyclo derivatives (for example containing a bridged piperidyl group such as a tropyl, pseudotropyl, homotropyl or quinuclidinyl group) and benzoic acid derivatives (for example benzoates and benzamides).

Preferred $5HT_3$ receptor antagonists for use according to the present invention are the compounds disclosed in published European Patent Application Nos. 306323 and 353983, and in published UK Patent Application No. 2202530.

Particular compounds having antagonist activity at $5HT_3$ receptors for use according to the present invention are:

Indole-[3α-(8-methyl-8-azabicyclo[3.2.1]octyl]-3-carboxylate; (ICS 205-930)
endo-N-(9-Methyl-9-azabicyclo[3,3,1]non-3-yl)-1-methyl-indazole-3-carboxamide; (BRL 43694)
3,5-Dichloro-[3α-(8-methyl-8-azabicyclo[3.2.1]octyl)]benzoate; (MDL 72222)
3,5-Dimethyl-[3α-(8-methyl-8-azabicyclo[3.2.1]octyl)]benzoate; (MDL 72422)
(±)-endo-4-amino-5-chloro-2-methoxy-N-(1-azabicyclo[3.3.3]non-4-yl) benzamide; (BRL 24924)
Indole-[5-(2-methyl-2-azabicyclo[2.2.2]octyl]-3-carboxylate;
1H-indol-3-yl-carboxylic acid (3R*, 4S*)-1-azabicyclo[2.2.1]hept-3-yl ester;
1H-indolyl-3-carboxylic acid 2S-(1-methyl-2-pyrrolidinylmethyl) ester.

A preferred $5HT_3$ receptor antagonist for use according to the invention is 1,2,3,9-tetrahydro-9-methyl-3-[(2-methyl-1H-imidazol-1-yl) methyl]-4H-carbazol-4-one which may be represented by the formula (II)

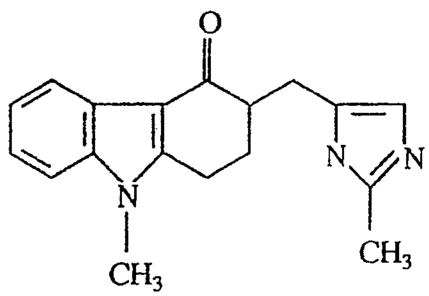

(II)

and physiologically acceptable salts and solvates thereof. The compound is disclosed inter alia in UK Patent Specification No. 2153821A and is described as a potent and selective antagonist of 5-hydroxytryptamine (5HT) at 'neuronal' 5HT receptors of the type located on terminals of primary afferent nerves and which are also present in the central nervous system. Receptors of this type are now designated $5HT_3$ receptors.

Compound (II) for use according to the invention may be administered as the free base or a physiologically acceptable salt or solvate thereof. Suitable salts include acid addition salts formed with inorganic acids such as hydrochlorides, hydrobromides, phosphates and sulphates and with organic acids such as tartrates, maleates, fumarates, succinates and sulphonates. A solvate may be, for example, a hydrate. A preferred form of the compound (II) for use according to the invention is the hydrochloride, particularly in hydrated form, for example the dihydrate.

Compounds for use according to the invention may be administered simultaneously or sequentially and, when administration is sequential, either the compound of formula (I) or the compound having antagonist activity at $5HT_3$ receptors may he administered first.

Compounds for use according to the invention may be administered as the raw material but the active ingredients are preferably provided in the form of pharmaceutical formulations.

The active ingredients may be used either as separate formulations or as a single combined formulation. When combined in the same formulation it will be appreciated that the two compounds must be stable and compatable with each other and the other components of the formulation. When formulated separately they may be provided in any convenient formulation, conveniently in such manner as are known for such compounds in the art.

Accordingly in a further aspect of the invention we provide a pharmaceutical composition which comprises the compound of formula (I) or a physiologically acceptable salt or solvate thereof and a compound having antagonist activity at $5HT_3$ receptors or a physiologically acceptable salt or solvate thereof formulated for administration by any convenient route. Such compositions are preferably in a form adapted for use in medicine, in particular human medicine, and can conveniently be formulated in conventional manner using one or more pharmaceutically acceptable carriers or excipients.

Compositions according to the invention may for example be formulated for oral, sub-lingual buccal, parenteral, rectal or intranasal administration or in a form suitable for administration by inhalation or insufflation. Preferably such compositions will be formulated for oral administration. It will be appreciated that when the two active ingredients are administered independently, each may be administered by different means.

For oral administration, the pharmaceutical compositions may take the form of, for example, tablets or capsules prepared by conventional means with pharmaceutically acceptable excipients such as binding agents (e.g. pregelatinised maize starch, polyvinylpyrrolidone or hydroxypropyl methylcellulose); fillers (e.g. lactose, microcrystalline cellulose or calcium phosphate); lubricants (e.g. magnesium stearate, talc or silica); disintegrants (e.g. potato starch or sodium starch glycollate); or wetting agents (e.g. sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (e.g. sorbitol syrup, methyl cellulose or hydrogenated edible fats); emulsifying agents (e.g. lecithin or acacia); non-aqueous vehicles (e.g. almond oil, oily esters or ethyl alcohol); and preservatives (e.g. methyl or propyl-p-hydroxybenzoates or sorbic acid).

For buccal administration the compositions may) take the form of tablets or lozenges formulated in conventional manner.

Compositions may be formulated for parenteral administration by injection, conveniently intravenous or subcutaneous injection, for example by bolus injection or continuous intravenous infusion. Formulations for injection may be presented in unit dosage form e.g. in ampoules or in multi-dose containers, with an added preservative.

The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use.

For rectal administration the compositions may be formulated as suppositories or retention enemas, e.g. containing conventional suppository bases such as cocoa butter or other glyceride.

Tablets for sub-lingual administration may be formulated in a similar manner to those for oral administration.

For intranasal administration the compositions may be presented as a liquid spray or in the form of drops.

For administration by inhalation compounds for use according to the invention are conveniently delivered in the form of an aerosol spray presentation from pressurised packs or a nebuliser, with the use of a suitable propellant, e.g. dichlorodifluoromethane, trichlorofluoromethane, dichlorotetrafluoroethane, carbon dioxide or other suitable gas. In the case of a pressurised aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

Pharmaceutical compositions according to the invention may be prepared by conventional techniques. When combined in the same formulation for example, the compound of formula (I) or a physiologically acceptable salt or solvate thereof and the compound having antagonist activity at $5HT_3$ receptors or physiologically acceptable salt or solvate thereof may be admixed together, if desired, with suitable excipients. Tablets may be prepared, for example, by direct compression of such a mixture. Capsules may be prepared, for example by filling the blend together with suitable excipients into gelatin capsules, using a suitable filling machine.

Compositions for use according to the invention may, if desired, be presented in a pack or dispenser device which may contain one or more unit dosage forms containing the active ingredients. The pack may, for example, comprise metal or plastic foil, such as a blister pack. Where the compounds are intended for administration as two separate compositions these may be presented, for example, in the form of a twin pack.

It will be appreciated that the dose at which the compound of formula (I) and the compound having antagonist activity at $5HT_3$ receptors is administered will depend on the age and condition of the patient and the frequency and route of administration. The active ingredients may conveniently be presented in unit dose form.

A proposed dose of the compound of formula (I) for administration to man (of approximately 70kg body weight) is 0.1mg to 100mg per unit dose which may be administered for example 1 to 4 times per day.

The compound having antagonist activity at $5HT_3$ receptors may conveniently be administered at doses within the normal range taught in the art at which the compounds are therapeutically effective. For example, a proposed dose of the compound of formula (II) for use according to the invention is 0.05 to 25 mg, preferably 0.05 to 20mg, most preferably 0.1 to 10mg per unit dose, expressed as the weight of free base The unit dose may be administered, for example, from 1 to 4 times per day.

The following examples illustrate pharmaceutical compositions according to the invention, containing 3-[2-(dimethylamino)ethyl]-N- methyl-1H-indole-5-methanesulphonamide succinate salt (1:1) (Compound A) and 1,2,3,9-tetrahydro-9-methyl-3-[(2-methyl-1H-imidazol-1-yl)methyl]-4H-carbazol-4-one hydrochloride dihydrate (Compound B) as the active ingredients. Other physiologically acceptable salts and/or solvates of the compound of formula (I) and other compounds having antagonist activity at $5HT_3$ receptors may be formulated in a similar manner.

TABLETS FOR ORAL ADMINISTRATION

Tablets may be prepared by normal methods such as direct compression or wet granulation.

| Direct Compression Tablet | mg/tablet |
|---|---|
| Compound A | 140.00 * |
| Compound B | 10.00 ** |
| Microcrystalline Cellulose NF | 35.00 |
| Anhydrous Lactose NF | 160.00 |
| Magnesium Stearate BP | 1.5 |
| Croscarmellose Sodium NF | 3.5 |
| Compression weight | 350.00 |

* 140mg Compound A is equivalent to 100mg free base

** 10mg Compound B is equivalent to 8mg free base

Compounds A and B, microcrystalline cellulose, anhydrous lactose and croscarmellose sodium are sieved and blended in a suitable mixer. the magnesium stearate is sieved and blended with the active mix. The resultant mix is compressed into tablets using suitable tablet punches.

| Wet Granulation Tablet | mg/tablet |
|---|---|
| Compound A | 140.0 |
| Compound B | 10.0 |
| Lactose BP | 60.0 |
| Pregelatinised Maize Starch BP | 35.0 |
| Croscarmellose Sodium NF | 3.5 |
| Magnesium Stearate BP | 1.5 |
| Compression weight | 350.0 |

Compounds A and B, lactose and pregelatinised starch are sieved and blended together and the resultant mix granulated with water. The wet mix is dried and milled. the croscarmellose sodium and magnesium stearate are sieved and blended with the dried granules. The resultant mix is compressed into tablets using suitable tablet punches.

CAPSULES

| | mg/capsule |
|---|---|
| Compound A | 140.0 |
| Compound B | 10.0 |
| Microcrystalline Cellulose NF | 46.0 |
| Magnesium Stearate BP | 4.0 |
| Fill Weight | 200.0 |

The active ingredients are seived and blended with the microcrystalline cellulose. The magnesium stearate is sieved and blended with the active mix. The mix is filled into hard gelatin capsules of suitable size.

**Claims**

1. A pharmaceutical composition comprising 3-[2-(dimethylamino)ethyl]-N-methyl-1H-indole-5-methanesulphonamide or a physiologically acceptable salt or solvate thereof and a compound having antagonist activity at $5HT_3$ receptors.

2. A pharmaceutical composition as claimed in Claim 1 wherein the compound having antagonist activity

at $5HT_3$ receptors is selected from :

Indole-[3α-(8-methyl-8-azabicyclo[3.2.1]octyl]-3-carboxylate; (ICS 205-930)
endo-N-(9-methyl-9-azabicyclo[3,3,1]non-3-yl)-1-methyl-indazole-3-carboxamide; (BRL 43694)
3,5-dichloro-[3α(8-methyl-8-azabicyclo[3.2.1]octyl)]benzoate; (MDL 72222)
3,5-dimethyl-[3α(8-methyl-8-azabicyclo[3.2.1]octyl)]benzoate; (MDL 72422)
(±)-endo-4-amino-5-chloro-2-methoxy-N-(1-azabicyclo[3.3.3]non-4-yl) benzamide; (BRL 24924)
Indole-[5-(2-methyl-2-azabicyclo [2.2.2]octyl]-3-carboxylate;
1H-indol-3-yl-carboxylic acid (3R*, 4S*)-1-azabicyclo[2.2.1]hept-3-yl ester; and
1H-indolyl-3-carboxylic acid 2S-(1-methyl-2-pyrrolidinylmethyl) ester; and physiologically acceptable salts and solvates thereof.

3. A pharmaceutical composition as claimed in Claim 1 wherein the compound having antagonist activity at $5HT_3$ receptors is selected from the compounds disclosed in published European Patent Application Nos. 306323 and 353983, and in published United Kingdom Patent Specification No. 2202530, and physiologically acceptable salts and solvates thereof.

4. A pharmaceutical composition as claimed in Claim 1 wherein the compound having antagonist activity at $5HT_3$ receptors is 1,2,3,9-tetrahydro-9-methyl-3-[(2-methyl-1H-imidazol-1-yl)methyl]-4H-carbazol-4-oneor a physiologically acceptable salt or solvate thereof.

5. A pharmaceutical composition as claimed in Claim 4 wherein the 1,2,3,9-tetrahydro-9-methyl-3-[2-methyl-1H-imidazol-1-yl)methyl]-4H-carbazol-4-one is present in the form of a hydrochloride salt.

6. A pharmaceutical composition as claimed in Claim 5 wherein the 1,2,3,9-tetrahydro-9-methyl-3-[(2-methyl-1H-imidazol-1-yl)methyl]-4H-carbazol-4-one is present in the form of the hydrochloride dihydrate.

7. A pharmaceutical composition as claimed in any of Claims 1 to 6 wherein the 3-[2-(dimethylamino)-ethyl]-N-methyl-1H-indole-5-methanesulphonamide is present in the form of the (1:1) succinate salt.

8. A pharmaceutical composition as claimed in any of Claims 1 to 7 in unit dose form containing 0.1 to 100mg of 3-[2-(dimethylamino)ethyl]-N-methyl-1H-indole-5-methanesulphonamide.

9. A pharmaceutical composition as claimed in any of Claims 4 to 8 in unit dose form containing 0.05 to 25mg of 1,2,3,9-tetrahydro-9-methyl-3-[(2-methyl-1H-imidazol-1-yl)methyl]-4H-carbazol-4-one.

10. A pharmaceutical composition as claimed in any of Claims 1 to 9 in a form adapted for oral administration.

11. The use of 3-[2-(dimethylamino)ethyl]-N-methyl-1H-indole-5-methanesulphonamide or a physiologically acceptable salt or solvate thereof together with a compound having antagonist activity at $5HT_3$ receptors for the manufacture of a medicament for the treatment of conditions associated with cephalic pain and the alleviation of symptoms associated therewith.

Claims for the following Contracting States: ES and GR

1. A method of manufacture of a pharmaceutical composition comprising 3-[2-(dimethylamino)ethyl]-N-methyl-1H-indole-5-methanesulphonamide or a physiologically acceptable salt or solvate thereof and a compound having antagonist activity at $5HT_3$ receptors, which method comprises processing the components by conventional techniques to form a pharmaceutical composition.

2. A method as claimed in Claim 1 wherein the compound having antagonist activity at $5HT_3$ receptors is selected from :

Indole-[3α-(8-methyl-8-azabicyclo[3.2.1]octyl]-3-carboxylate; (ICS 205-930)
endo-N-(9-methyl-9-azabicyclo[3,3,1]non-3-yl)-1-methyl-indazole-3-carboxamide; (BRL 43694)
3,5-dichloro-[3α-(8-methyl-8-azabicyclo[3.2.1]octyl)]benzoate; (MDL 72222)

3,5-dimethyl-[3α-(8-methyl-8-azabicyclo[3.2.1]octyl)]benzoate; (MDL 72422)
(±)-endo-4-amino-5-chloro-2-methoxy-N-(1-azabicyclo[3.3.3]non-4-yl) benzamide; (BRL 24924)
Indole-[5-(2-methyl-2-azabicyclo [2.2.2]octyl]-3-carboxylate;
1H-indol-3-yl-carboxylic acid (3R*, 4S*)-1-azabicyclo[2.2.1]hept-3-yl ester; and
1H-indolyl-3-carboxylic acid 2S-(1-methyl-2-pyrrolidinylmethyl) ester; and physiologically acceptable salts and solvates thereof.

**3.** A method as claimed in Claim 1 wherein the compound having antagonist activity at $5HT_3$ receptors is selected from the compounds disclosed in published European Patent Application Nos. 306323 and 353983, and in published United Kingdom Patent Specification No. 2202530, and physiologically acceptable salts and solvates thereof.

**4.** A method as claimed in Claim 1 wherein the compound having antagonist activity at $5HT_3$ receptors is 1,2,3,9-tetrahydro-9-methyl-3-[(2-methyl-1H-imidazol-1-yl)methyl]-4H-carbazol-4-oneor a physiologically acceptable salt or solvate thereof.

**5.** A method as claimed in Claim 4 wherein the 1,2,3,9-tetrahydro-9-methyl-3-[(2-methyl-1H-imidazol-1-yl)-methyl]-4H-carbazol-4-one is present in the form of a hydrochloride salt.

**6.** A method as claimed in Claim 5 wherein the 1,2,3,9-tetrahydro-9-methyl-3-[(2-methyl-1H-imidazol-1-yl)-methyl]-4H-carbazol-4-one is present in the form of the hydrochloride dihydrate.

**7.** A method as claimed in any of Claims 1 to 6 wherein the 3-[2-(dimethylamino)ethyl]-N-methyl-1H-indole-5-methanesulphonamide is present in the form of the (1:1) succinate salt.

**8.** A method as claimed in any of Claims 1 to 7 wherein the pharmaceutical composition is in unit dose form containing 0.1 to 100mg of 3-[2-(dimethylamino)ethyl]-N-methyl-1H-indole-5-methanesulphonamide.

**9.** A method as claimed in any of Claims 4 to 8 wherein the pharmaceutical composition is in unit dose form containing 0.05 to 25mg of 1,2,3,9-tetrahydro-9-methyl-3-[(2-methyl-1H-imidazol-1-yl)methyl]-4H-carbazol-4-one.

**10.** A method as claimed in any of Claims 1 to 9 wherein the pharmaceutical composition is in a form suitable for oral administration.

European Patent Office

# EUROPEAN SEARCH REPORT

Application Number

EP 90 31 3508

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y,D | GB-A-2 162 522 (GLAXO GROUP LTD UK)<br>* Page 19, lines 8-9, claim 9; page 2, lines 10-15 *<br>- - - | 1-11 | A 61 K 31/415 //<br>(A 61 K 31/415<br>A 61 K 31:40 ) |
| Y,D | GB-A-2 202 530 (GLAXO GROUP LTD U.K.)<br>* Page 9, lines 20-26 *<br>- - - | 1-11 | |
| Y,D | EP-A-0 306 323 (GLAXO GROUP LTD)<br>* Page 5, lines 19-24 *<br>- - - | 1-11 | |
| Y,D,P | EP-A-0 353 983 (GLAXO GROUP LTD)<br>* Page 5, line 65 - page 6, line 6 *<br>- - - - - | 1-11 | |

| TECHNICAL FIELDS SEARCHED (Int. Cl.5) |
|---|
| A 61 K |

The present search report has been drawn up for all claims

| Place of search | Date of completion of search | Examiner |
|---|---|---|
| The Hague | 19 March 91 | BRINKMANN C. |

CATEGORY OF CITED DOCUMENTS

- X: particularly relevant if taken alone
- Y: particularly relevant if combined with another document of the same catagory
- A: technological background
- O: non-written disclosure
- P: intermediate document
- T: theory or principle underlying the invention
- E: earlier patent document, but published on, or after the filing date
- D: document cited in the application
- L: document cited for other reasons
- &: member of the same patent family, corresponding document